# EUROPEAN PATENT SPECIFICATION

(11) **EP 1 651 753 B1**
(45) Date of publication and mention of the grant of the patent: **16.03.2011**
(21) Application number: 04733702.7
(22) Date of filing: 18.05.2004
(51) Int. Cl.: C12N 1/16, C12P 19/04

(54) **NOVEL CANDIDA TROPICALIS CJ-FID(KCTC 10457BP) AND MANUFACTURING METHOD OF XYLITOL THEREBY**
NEUES CANDIDA TROPICALIS CJ-FID(KCTC 10457BP) UND HERSTELLUNGSVERFAHREN FÜR XYLITOL DAMIT
NOUVEAU CANDIDA TROPICALIS CJ-FID (KCTC 10457BP) ET METHODE ASSOCIEE DE PRODUCTION DE XYLITOL

(30) Priority: 25.07.2003 KR 2003051593; 13.05.2004 KR 2004033733
(43) Date of publication of application: 03.05.2006
(73) Proprietor: CJ CheilJedang Corporation, Jung-gu Seoul 100-095 (KR)
(72) Inventor: KIM, Jung-hoon, Seoul 152-763 (KR); SOHN, Young-rok, Songpa-gu, Seoul 138-050 (KR); LEE, Woon-hwa, Anyang-si, Gyeonggi-do 431-080 (KR); PARK, Seung-won, Jinsanmaeul Samsung 7cha Apt., Gyeonggi-do 449-170 (KR); PARK, Kang-june, Incheon 406-130 (KR); LEE, Ki-chang, Incheon 400-103 (KR); LIM, Jae-kag, Seoul 137-040 (KR)
(74) Representative: Campbell, Neil Boyd
(86) International application number: PCT/KR2004/001172
(87) International publication number: WO 2005/010171

(56) References cited:
- JP-A- 60 145 095
- KR-A- 99 065 347
- KR-A- 99 086 862
- US-A- 5 998 181
- CARVALHO WALTER ET AL: "Improvement in xylitol production from sugarcane bagasse hydrolysate achieved by the use of a repeated-batch immobilized cell system." ZEITSCHRIFT FÜR NATURFORSCHUNG. C, JOURNAL OF BIOSCIENCES. 2002 JAN-FEB, vol. 57, no. 1-2, January 2002 (2002-01), pages 109-112, XP002410232 ISSN: 0341-0382
- CARVALHO WALTER ET AL: "Metabolic behavior of immobilized Candida guilliermondii cells during batch xylitol production from sugarcane bagasse acid hydrolyzate" BIOTECHNOLOGY AND BIOENGINEERING, vol. 79, no. 2, 20 July 2002 (2002-07-20), pages 165-169, XP002410233 ISSN: 0006-3592
- SOLANGE I M ET AL: "Hydrolysate detoxification with activated charcoal for Xylitol production by Candida guilliermondii" BIOTECHNOLOGY LETTERS, KEW, SURREY, GB, vol. 23, 2001, pages 1681-1684, XP002985879 ISSN: 0141-5492
- DOMINGUEZ JOSE MANUEL ET AL: "Xylitol production from wood hydrolyzates by entrapped Debaryomyces hansenii and Candida guilliermondii cells" APPLIED BIOCHEMISTRY AND BIOTECHNOLOGY, vol. 81, no. 2, August 1999 (1999-08), pages 119-130, XP009075829 ISSN: 0273-2289
- CARVALHO W. ET AL.: 'Use of immobilized candida yeast cells for xylitol production from sugarcane bagasse hydrolysate: cell immobilization conditions' APPL. BIOCHEM. BIOTECHNOL. vol. 98, no. 100, 2002, pages 489 - 496, XP008077989

<110> CJ Corp. ,
   KIM, Jung-hoon
   SOHN, Young-rok
   LEE, Woon-hwa
   PARK, Seung-won
   PARK, Kang-june
   LEE, Ki-chang
   LIM, Jae-kag
<120> Novel Candida tropicalis CJ-FID(KCTC 104576P) and Manufacturing Method of Xyl Itol thereby
<130> PP04-0114
<150> KR10-2003-0051593
   <151> 2003-07-28
<150> KR10-2004-0033733
   <151> 2004-05-13
<160> 1
<170> Kopatent In 1.71
<210> 1
   <211> 576
   <212> DNA
   <213> Candida tropicalis
<400> 1
   ataccaacag ggattgcctt agtagcggcg agtgaagcgg caaaagctca aatttgaaat      60
   ctggctcttt cagagtccga gttgtaattt gaagaaggta tctttgggtc tggctcttgt     120
   ctatgtttct tggaacagaa cgtcacagag ggtgagaatc ccgtgcgatg agatgatcca     180
   ggcctatgta aagttccttc gaagagtcga gttgtttggg aatgcagctc taagtgggtg     240
   gtaaattcca tctaaagcta aatattggcg agagaccgat agcgaacaag tacagtgatg     300
   gaaagatgaa aagaactttg aaaagagagt gaaaaagtac gtgaaattgt tgaaagggaa     360
   gggcttgaga tcagacttgg tattttgtat gttacttctt cgggggtggc ctctacagtt     420
   tatcgggcca gcatcagttt gggcggtagg agaattgcgt tggaatgtgg cacggcttcg     480
   gttgtgtgtt atagccttcg tcgatactgc cagcctagac tgaggactgc ggtttatacc     540
   taggatgttg gcataatgat cttaagtcgc ccgtct                               576

## Description

### Technical Field

The present invention relates to a novel *Candida tropicalis* CJ-FID and a method for producing xylitol using the same. More particularly, the present invention relates to a novel *Candida tropicalis* CJ-FID derived from honey and a method for producing high purity xylitol with high yield and productivity, which includes culturing the *Candida tropicalis* CJ-FID itself or an alginate-immobilized *Candida tropicalis* CJ-FID in a fermentation medium containing high concentration xylose and sucrose to obtain a xylitol-containing culture, followed by purification with an activated charcoal column and an anion column, to finally obtain powdery xylitol.

### Background Art

Xylitol is a sugar alcohol produced by conversion of a reducing end group of xylose, which is a five-carbon sugar, to an alcohol group by hydrogenation, and is found in small quantities in natural plants such as fruits, vegetables, and mushrooms. Xylitol is also known as an intermediate in mammalian carbohydrate metabolism. Xylitol is more stable than other sugars because of its chemical structure, has high affinity for water, and does not undergo a browning reaction.

Xylitol has sweetness equal to sucrose but contains a low-calorie and does not cause elevation of a blood sugar level or tooth decay. In this respect, xylitol has become very popular as a sugar substitute (McNutt K., A. Sentki. 1996. Sugar replacers: a growing group of sweeteners in the United States. Nutr. Today. 31 (6) : 255-261). Xylitol is absorbed in the human intestinal tract by active diffusion. However, since an absorption rate is quite slow, xylitol is incompletely absorbed by the intestinal tract and excreted out of the body. Absorbed xylitol is partially broken down by intestinal microorganisms and only a small fraction is utilized in the human body. In this respect, xylitol falls under the category of a low-calorie sweetener.

In particular, since it does not require insulin when being absorbed into cell tissues, xylitol is used as a sugar substitute for diabetic patients. Furthermore, because of its sweetness equivalent to sucrose and its negative heat of dissolution, xylitol imparts a cool and fresh sensation in the oral cavity. Therefore, xylitol is used in various food industries including confectionery products and chewing gums. In addition, xylitol can prevent cavities by inhibiting the growth of *Streptococcus mutans* which is responsible for tooth decay, and thus, is used as a material of toothpaste.

Xylitol is found in vegetables or fruits, but extraction of xylitol from these sources is uneconomical due to its low concentration. In this respect, the chemical production of xylitol has been mainly used. According to the chemical production method, xylitol is produced by chemical reduction of xylose which is a hydrolysate of hemicellulose present in woods, ice straw, millet, etc., under high temperature and high pressure conditions. However, separation and purification of xylose or xylitol from hydrolysates of other polymer sugars derived fromhemicellulose fractions are difficult and the yield of xylose or xylitol is as low as 50-60%. Furthermore, there arise problems such as a risk of high temperature and high pressure reaction and waste disposal due to use of acid or alkali.

There is also known xylitol production using microorganism as a biocatalyst for conversion of xylose in a xylose-containing medium into xylitol. That is, in microorganism, xylose in a xylose-containing medium is transported across the cell membrane and then is converted to xylitol by NADPH coenzyme-dependent xylose reductase (XR). Abundantly accumulated xylitol is released from the cells of microorganism. The xylitol production using microorganism has advantages in that separation of xylitol fromxylose is not required due to complete consumption of xylose after fermentation and mild conditions such as room temperature and atmospheric pressure are used. However, since it is difficult to efficiently and selectively remove byproducts such as organic acids and glycerol, medium ingredients, and substances derived from microorganism, which are generally contained in a fermentation medium, purification of high purity xylitol is difficult, resulting in low yield.

Korean Patent Laid-Open Publication No. 10-2001-49918 discloses a method for producing xylitol from arabitol using *Gluconobacter oxydans* ATCC 621. According to the disclosure in the patent document, xylitol is purified from 200 ml of a 45 g/L xylitol solution finally produced. However, this document does not disclose an accurate puri f icat ion yield result. Furthermore, since xylitol is produced from a low concentration (50g/L) xylitol solution, high concentration and efficiency xylitol production is not ensured, which renders mass production difficult. Recently, to solve these problems, many studies of separation of a new yeast strain and a xylitol production method using the new yeast strain have been done.

### Disclosure of the Invention

In view of these problems, the present disclosure provides a novel *Candida tropicalis* CJ-FID. The present invention also provides a method for producing high purity xylitol with high yield and productivity.

The above and other objects of the present invention can be accomplished by embodiments of the present invention as will be described hereinafter.

Therefore, according to an aspect of the present disclosure, there is provided a xylitol-producing *Candida tropicalis* CJ-FID (KCTC 10457BP).

According to another aspect of the present invention, there is provided an alginate bead prepared by adding *Candida tropicalis* CJ-FID (UCTC 10457BP) to an aqueous alginic acid solution with stirring to obtain a uniform solution and dripping the uniform solution to a calcium salt.

According to still another aspect of the present invention, there is provided a method for producing xylitol, including preparing a xylitol-producing fermentation medium containing xylose and sucrose, inoculating a *Candida tropicalis* CJ-FID-containing alginate bead onto the fermentation medium, and fermenting the fermentation medium in a fermenter.

The method may further include purifying with an activated charcoal column of a xylitol-containing fermentation culture produced by the fermentation, purifying with an anion column of a xylitol solution produced by the purification with the activated charcoal column, and preparing a xylitol powder from a xylitol solution produced by the purification with the anion column.

Hereinafter, the present invention will be described in more detail.

### Separation of xylitol-producing yeast strain

A natural honey sample was taken from a domestic honey-producing district and appropriately diluted in a sterilized tube. The diluted honey sample was cultured in a plate medium containing 200-400g/L of xylose, 5 g/L of a yeast extract, 5 g/L of peptone, and 15-20 g/L of agar at 30°Cto obtain single colonies formed by yeast strains growing rapidly under the above growth conditions.

### Identification of xylitol-producing yeast strains

Among these colonies, yeast strains with excellent sugar tolerance and xylitol productivity under the above growth conditions were selected and their morphological and physiological characteristics were analyzed. As a result of 26S rRNA sequencing, it was found that the yeast strains have a nucleotide sequence as set forth in SEQ ID NO: 1. The yeast strains were designated as *Candida tropicalis* CJ-FID and deposited at Gene Bank of Korea Research Institute of Bioscience and Biotechnology (Accession No.: KCTC 10457BP).

### Evaluation of xylitol productivity

To evaluate the xylitol productivity of the selected yeast strains, the selected yeast strains were inoculated onto a 50 mL seed medium containing 2 0 g/L of xylose, 5 g/L of yeast extract, 5 g/L of peptone, and 2 g/L of sucrose, in a 250 mL flask, and then shaking culture was performed at 200 rpm and 30°Cfor 12 hours. Then, 5% of the seed culture was inoculated onto a 100 mL main medium containing 100g/Lof xylose, 5 g/Lof yeast extract, 5g/L of peptone, and 10g/L of sucrose, in a 500 mL flask, and then shaking culture was performed at 150 rpm and 30°Cfor 42 hours. Culture samples were taken from the resultant culture at predetermined intervals and then xylitol production in each culture sample was determined.

### Xylose and xylitol analysis

For analysis of xylose and xylitol, the culture samples taken in the previous Section were centrifuged at 12,000 rpm to remove cells. Supernatants were analyzed using Refractive Index Detector (Shimadzu C-R6A, Japan) for high-performance liquid chromatography (HPLC, Shimadzu, Japan) equipped with Kromasil 100-10 NH2 column (Denmark). At this time, a solvent was 90% acetonitrile and a flow rate was 2.0 mL/min. Yeast cell density was estimated by measuring an optical density at 600 nm using a tubidimeter and comparing the value to a previously prepared standard curve in which optical density is related to cell dry weight.

### Immobilization of Candida tropicalis CJ-FID

To prepare immobilized cells, an alginic acid solution (3%, w/v) used in foods was prepared. Then, the same concentration of a cell concentrate was added thereto and stirred. The resultant solution was dripped into a 100 mM calcium chloride solution in a bead generator to form alginate beads. The alginate beads thus formed were stored at 4°Cfor 12 hours to increase the strength of the alginate beads for subsequent experiments.

### Xylitol fermentation by Candida tropicalis CJ-FID

A fermentation medium containing xylose, 0.1-10g/Lof yeast extract, 0.1-10 g/L of peptone, and 0.1-50 g/L of sucrose was introduced into a fermenter. Then, the *Candida tropicalis* CJ-FID (KCTC10457BP) itself or the alginate-immobilized *Candida tropicalis* CJ-FID was inoculated onto the fermentation medium and cultured under aeration at a rate of 0.5-2 vvm at 200-300 rpm and 25-35°C for 60 to 100 hours. After the fermentation, the fermentation culture was centrifuged to remove cells and xylitol was recovered from supernatants.

Preferably, xylose is contained in the fermentation medium in an amount of 50 to 200 g/L, and more preferably in an amount of 80 to 150 g/L. If the content of xylose is less than 50 g/L, the yield of xylitol may be lowered. On the other hand, if it exceeds 200g/L, a productivity enhancement maybe insignificant. For productivity enhancement, the fermentation medium may be supplemented with 0.1-5 g/L of potassium phosphate (KH₂PO₄) and 0.5-5 g/L of magnesium sulfate (MgSO₄).

### Purification of xylitol fermentation culture

To produce high purity xylitol powders from the fermentation culture obtained in the previous Section, 300 g of an activated charcoal was packed into a column (60 cm in height x 10 cm in diameter) and then the fermentation culture was allowed to pass through the column to obtain off-flavored and discolored xylitol-containing *fractions.* The obtained xylitol-containing fractions were allowed to pass through an anion column (60 cm in height x 10 cm in diameter) packed with anionic resin to remove impurities. The resultant xylitol-containing fractions were analyzed by HPLC. As a result, pure xylitol was present in the xylitol-containing *fractions*.

The xylitol-containing *fractions* were concentrated to more than 800 g/L in a concentrator and crystallized at 4°C followed by addition of an alcoholic ethanol with stirring, to give fine xylitol crystals. The xylitol crystals were filtered in vacuum to partially remove ethanol and water. Finally, residual ethanol and water were removed by a vacuum dryer to give xylitol powders.

### Brief Description of the Drawings

FIG. 1 is a graph that illustrates xylitol productivity with respect to an initial xylose concentration.
FIG. 2 is a graph that illustrates xylitol productivity with respect to an initial cell density.
FIG. 3 is a graph that illustrates xylitol productivity and yield with respect to the density of alginate-immobilized cells.
FIG. 4 is a graph that illustrates xylitol productivity and yield with respect to the mass of alginate beads.
FIG. 5 is a graph that illustrates xylitol productivity and yield with respect to seed culture time of yeast cells to be immobilized with alginate.
FIG. 6 is a graph that illustrates xylitol productivity and yield with fermentation time of alginate-immobilized yeast cells in a 2L fermenter set to optimal fermentation conditions.
FIG. 7 is a graph that illustrates xylitol productivity and yield with fermentation time of alginate-immobilized yeast cells in a 17 L fermenter set to optimal fermentation conditions.
FIG. 8 is a graph that illustrates a change in xylitol concentration for xylitol-containing fractions obtained by application of a xylitol-containing culture to an activated charcoal column.
FIG. 9 is a graph that illustrates a change in xylitol concentration for the xylitol-containing fractions obtained by application of the fractions of FIG. 8 to an anion column.
FIG. 10 is a high-performance liquid chromatogram (HPLC) for the fractions of FIG. 9.
FIG. 11 is a graph that illustrates xylitol crystallization yield of the fractions of FIG. 9 concentrated to different concentrations.

### Best mode for carrying out the Invention

Hereinafter, the present invention will be described more specifically by Examples. However, the following Examples are provided only for illustrations and thus the present invention is not limited to or by them.

### [Example 1] Xylitol productivity with respect to initial xylose concentration

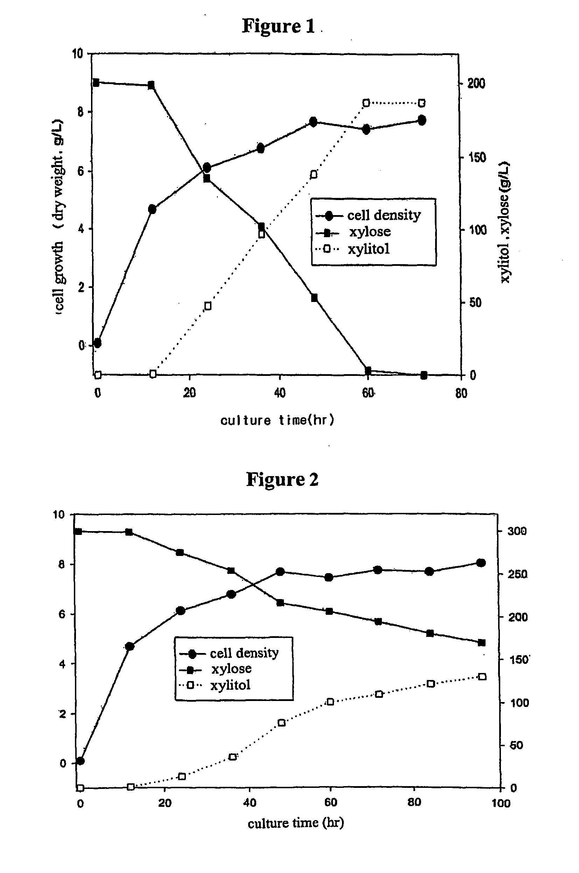

After performing a seed culture as described above, the seed culture was inoculated onto a 10 L xylitol-producing main medium containing 0.5 g/L of yeast extract, 5 g/L of peptone, 10-30 g/L of sucrose, and 100-300 g/L of xylose, and cultured ina 15 L fermenter (Korea Fermentation Co., Ltd.). Fermentation conditions were as follows: stirring speed of 250 rpm, aeration flow speed of 1.0 vvm, culture temperature of 30°C Xylitol productivity with respect to initial xylose concentration was measured and the results are shown in FIG. 1.

As showninFIG. 1, themaximumxylitol production was 187g/L. At this time, the production yield of xylitol was excellent by 93.5%. It can be seen that addition of sucrose increased the yield of xylitol.

### [Example 2] Xylitol productivity with respect to initial cell density

After performing a seed culture as described above, the seed culture was inoculated onto a 10 L xylitol-producing main medium containing 5 g/L of yeast extract, 5 g/L of peptone, 10 g/L of sucrose, and 100 g/L of xylose, and cultured in a 15 L fermenter (Korea Fermentation Co., Ltd.). Fermentation conditions were as follows: stirring speed of 250 rpm, aeration flow speed of 1.0 vvm, and culture temperature of 30°C. Xylitol productivity with respect to initial cell density was measured and the results are shown in FIG. 2.

As shown in FIG. 2, the maximum xylitol production was 132 g/L. At this time, the ratio of xylitol produced to xylose used was excellent by 98%. However, it can be seen that use of high concentration xylose retarded the production speed of xylitol.

### [Example 3] Sugar tolerance test for conventional xylitol-producing strains and Candida tropicalis CJ-FID (KCTC 10457BP)

The *candida tropicalis* CJ-FID according to the present invention and conventional xylitol-producing strains were evaluated for sugar tolerance and the evaluation results are presented in Table 1 below.

**Table 1: Results of sugar tolerance test for xylitol-producing yeast strains**

| Yeast strain | Xylose concentration (g/L) | | | |
|---|---|---|---|---|
| | 100 | 200 | 300 | 400 |
| *Candida tropicalis* KCTC 7101 | +++ | +++ | + | - |
| *Debaryomyces hanseni* KCTC 7128 | +++ | +++ | - | - |
| *Candida* tropicalis KCTC 7212 | +++ | +++ | - | - |
| *Candida parapsilosis* KCTC7653 | +++ | +++ | - | - |
| *Candida tropicalis* KCTC 7725 | +++ | +++ | + | - |
| *Candida tropicalis* KCTC 7901 | +++ | +++ | - | - |
| *Candida sp.* KCTC 17041 | +++ | +++ | - | - |
| *Candida shehata* KCCM 11895 | +++ | +++ | + | - |
| *Candida guilliermondii* KCCM 50050 | +++ | +++ | - | - |
| *Candida tropicalis* KCCM 50091 | +++ | +++ | + | - |
| *Candida mogii* KCCM 50199 | +++ | +++ | - | - |
| *Candida guilliermondii* KCCM 50631 | +++ | +++ | - | - |
| *Candida utilis* KCCM 50667 | +++ | +++ | - | - |
| *Candidatropicalis* CJ-FID KCTC 10457BP | +++ | +++ | ++ | + |

| | | | | |
|---|---|---|---|---|
| (+++ : excellent growth, ++ : good growth, + : slight growth, - : no growth) | | | | |

As can be seen from Table 1, the *Candida tropicalis* CJ-FID (KCTC 10457BP) of the present invention exhibited excellent xylose tolerance, as compared to currently known yeast strains. This means that the *Candida tropicalis* CJ-FID (KCTC 10457BP) of the present invention can produce xylitol in large scale in a high-concentration xylose-containing medium.

### [Example4] Evaluation of xylitol productivity with respect to density of alginate-immobilized cells

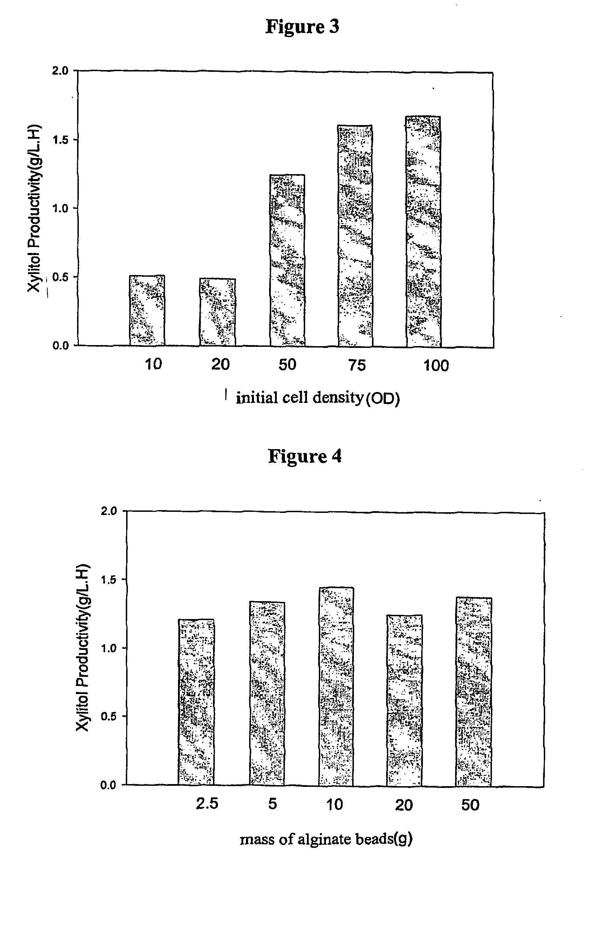

To evaluatexylitol productivity with respect to the density of alginate bead-entrapped *Candida tropicalis* CJ-FID yeast cells, after performing a seed culture as described above, alginate beads were prepared by mixing an alginate solution with the seed culture with varying the cell density (10 to 100 (OD value)) . Then, the alginate beads were inoculated onto 10 L xylitol-producing main media containing 5 g/L of yeast extract, 5 g/L of peptone, and 100 g/L of xylose, and cultured in a 15 L fermenter (Korea Fermentation Co., Ltd.). Fermentation conditions were as follows: stirring speed of 250 rpm, aeration flow speed of 1.0 vvm, culture temperature of 30°C. Xylitol productivity with respect to initial cell density was measured and the results are shown in FIG. 3.

As shown in FIG. 3, when the cell density was 10 to 20 (OD value), xylitol productivity was about 0.5g/L.H. At the cell density of more than 50 (OD value), xylitol productivity was more than 1.25g/L.H. At the cell density of 100 (OD value), xylitol productivity was maximal as 1.65g/L.H.

### [Example 5] Evaluation of xylitol productivity with respect to mass of alginate beads

Alginate beads prepared in the same manner as in Example 4 were inoculated with varying mass (2.5 to 50 g) onto xylitol-producing fermentation media in a 2 L fermenter and cultured under the same fermentation conditions as in Example 4. Xylitol productivity with respect to mass of the alginate beads was measured and the results are shown in FIG. 4.

As shown in FIG. 4, xylitol productivity was maintained constant regardless of change in mass of the alginate beads.

### [Example 6] Evaluation of xylitol productivity with respect to seed culture time

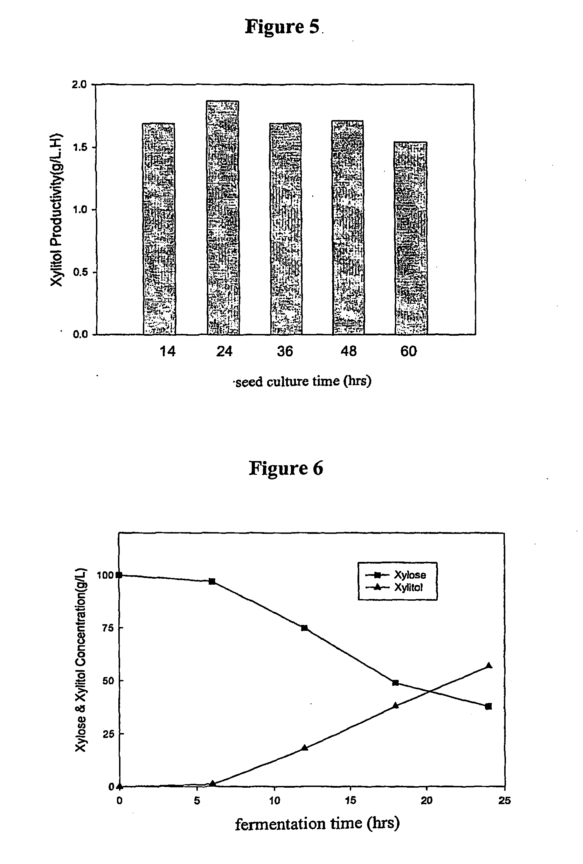

To evaluate xylitol productivity with respect to seed culture time of seed culture cells used for preparation of alginate beads, yeast strains were cultured in seed media with varying the culture time (12 to 60 hours) and then fermentation was performed under the same fermentation conditions as in Example 4. Xylitol productivity with respect to seed culture time was measured and the results are shown in FIG. 5.

As shown in FIG. 5, xylitol productivity was maintained constant regardless of seed culture time. However, the yeast strains that had been seed-cultured for 24 hours exhibited the highest xylitol productivity..

### [Example 7] Evaluation of xylitol productivity and yield with respect to repetitive use of immobilized cells

Alginate bead-immobilized *Candida tropicalis* CJ-FID cells were prepared and fermented in the same manner as in Example 4 except that the alginate beads were used repetitively (once to five times). Xylitol productivity and yields of the alginate bead-immobilized *Candida tropicalis* CJ-FID cells were evaluated by comparing with those of currently known alginate bead-immobilized *Candida guilliermondii* FTI 20037, and the results are presented in Table 2 below.

**Table 2: Xylitol productivity and yields with respect to repetitive use of immobilized yeast cells**

| Strain | Section | Repetitive number | | | | |
|---|---|---|---|---|---|---|
| | | 1 | 2 | 3 | 4 | 5 |
| *Candida* tropicalis CJ-FID (KCTC 110457BP) | Productivity (g/L.H) | 3.31 | 2.95 | 3.04 | 3.11 | 2.99 |
| | Yield (%) | 89.5 | 92.3 | 88.6 | 90.4 | 90.6 |
| *Candida guilliermondii* FTI 20037 | Productivity (g/L.H) | 0.5 | 0.59 | 0.6 | 0.6 | 0.58 |
| | Yield (%) | 53.2 | 59.5 | 62.6 | 62.4 | 61.4 |

As shown in Table 2, even when the immobilized cells were used five times, no reduction of xylitol productivity and yields was observed. In particular, the *Candida tropicalis* CJ-FID cells exhibited a five-fold increase for xylitol productivity and a more than 45% increase for xylitol yield, as compared to the *Candida guilliermondii* FTI 20037 cells.

### [Example 8] Xylitol productivity with respect to fermentation time

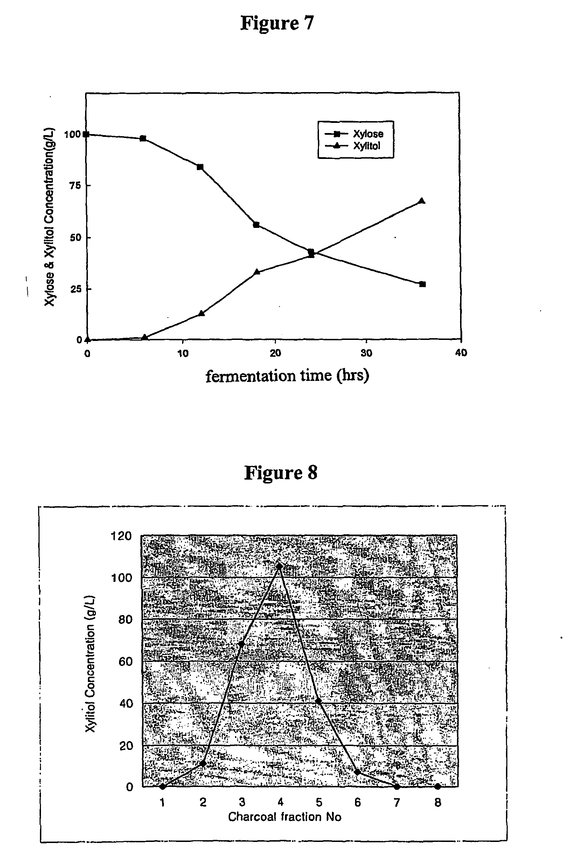

Alginate bead-immobilized *Candida tropicalis* CJ-FID cells were prepared and fermented in 2 L and 17 L fermenters in the same manner as in Example 4. Xylitol productivity was measured by varying the fermentation time and the results are shown in FIGS. 6 and 7.

As shown in FIGS. 6 and 7, xylitol concentration was increased from 6 hours of the fermentation. With respect to the fermentation in the 2L fermenter (FIG. 6), xylitol productivity was maximal at 24 hours of the fermentation. With respect to the fermentation in the 17 L fermenter, xylitol productivity was maximal at 36 hours of the fermentation.

### [Example 9] Purification of xylitol fermentation culture

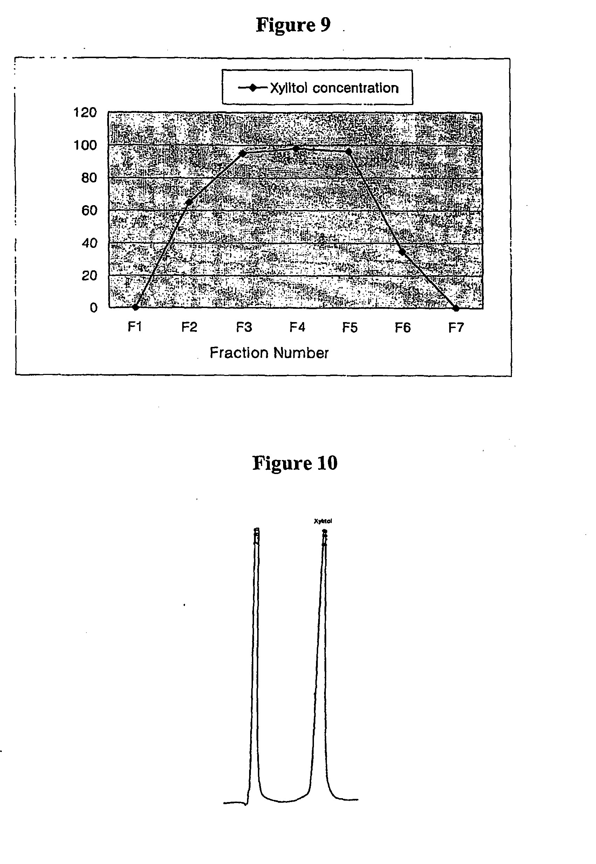

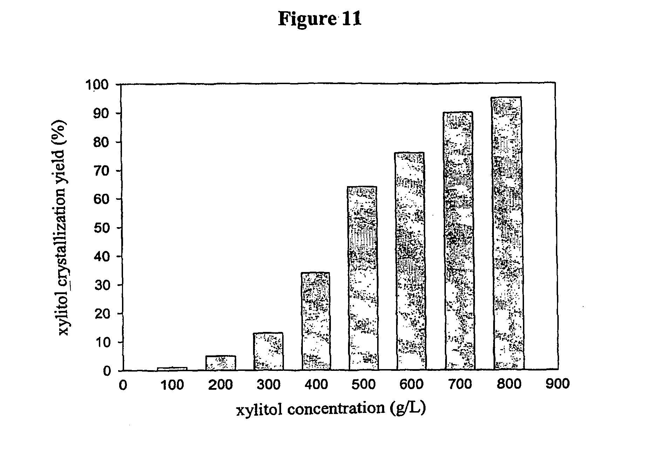

Fractions of a xylitol fermentation culture that had passed through an activated charcoal column and an anion column were measured for xylitol concentrations. The results are shown in FIGS. 8 and 9. The fractions of FIG. 9 were analyzed by high-performance liquid chromatography (HPLC) and the results are shown in FIG. 10. The fractions of FIG. 9 were concentrated to different concentrations and then xylitol crystallization yields with respect to the concentrations were measured. The results are shown in FIG. 11.

According to purification results using the activated charcoal column as shown in FIG. 8, a fraction 4 exhibited the highest xylitol concentration. According to purification results using the activated charcoal column and the anion column as shown in FIG. 9, fractions 3 through 5 exhibited the highest xylitol concentration. From the chromatogram of FIG. 10, it can be seen that the fractions that had passed through the activated charcoal column and the anion column contained highly purified xylitol. The purified xylitol solutions were crystallized to different concentrations. As the concentration of the xylitol solutions increased, the xylitol crystallization yield increased proportionally.

### [Example 10] Xylitol concentration and yield according to xylitol fermentation and purification

Xylitol concentrations and yields measured after the fermentation and the purification are summarized in Table 3 below.

**Table 3: Purification yields according topurificationprocesses of xylitol fermentation culture**

| Section | Xylitol concentration (g/L) | yield (%) |
|---|---|---|
| Xylitol fermentation culture (300 g/L) | 270 | 90 |
| Activated charcoal column | 270 | 90 |
| Anion column | 262 | 87.3 |
| Concentration | 257 | 84.7 |
| Crystallization | 244 | 80.4 |
| Powder formation | 230 | 76.4 |
| Final purification yield | 77.4 | |

As shown in Table 3, the final yield of xylitol after fermentation and purification was as high as 77.4% and very little xylitol loss during each purification process was observed.

### Industrial Applicability

As apparent from the above description, the novel *Candida tropicalis* CJ-FID (KCTC 10457BP) of the present invention and a xylitol production method using the same can produce highly pure xylitol with high yield and productivity under optimized culture condition and medium composition.
## Claims

1. An alginate bead comprising *Candida tropicalis* CJ-FID (KCTC 10457BP) prepared by adding the *Candida tropicalis* CJ-FID (KCTC 10457BP) to an aqueous alginic acid solution with stirring to obtain a uniform solution and dripping the uniform solution to a calcium salt.

2. A method for producing xylitol from xylose, comprising:
preparing a xylitol-producing fermentation medium containing xylose and sucrose;
inoculating the alginate bead of claim 1 onto the fermentation medium; and
fermenting the fermentation medium in a fermenter.

3. The method of claim 2, further comprising:
purifying with an activated charcoal column of a xylitol-containing fermentation culture produced by the fermentation;
purifying with an anion column of a xylitol solution produced by the purification with the activated charcoal column; and
preparing a xylitol powder from a xylitol solution produced by the purification with the anion column.

## Patentansprüche

1. Alginatkügelchen mit *Candida tropicalis* CJ-FID (KCTC 10457BP), das durch Zugeben des *Candida tropicalis* CJ-FID (KCTC 10457BP) zu einer wässerigen Alginsäurelösung unter Rühren zum Erhalten einer gleichmäßigen Lösung und Tropfen der gleichmäßigen Lösung auf ein Kalziumsalz hergestellt wird.

2. Verfahren zur Erzeugung von Xylit aus Xylose, das umfasst:
Herstellen eines Xylit erzeugenden Fermentationsmediums, das Xylose und Saccharose enthält;
Inokulieren des Alginatkügelchens von Anspruch 1 auf das Fermentationsmedium; und
Fermentieren des Fermentationsmediums in einem Fermenter.

3. Verfahren nach Anspruch 2, das ferner umfasst:
Reinigen einer Xylit enthaltenden Fermentationskultur, die durch die Fermentation erzeugt wird, mit einer Aktivkohlesäule;
Reinigen einer Xylitlösung, die durch die Reinigung mit der Aktivkohlesäule erzeugt wird, mit einer Anionensäule; und
Herstellen eines Xylitpulvers aus einer Xylitlösung, die durch die Reinigung mit der Anionensäule erzeugt wird.

## Revendications

1. Perle d'alginate comprenant *Candida tropicalis* CJ-FID (KCTC 10457BP), préparée en ajoutant le *Candida tropicalis* CJ-FID (KCTC 10457BP) à une solution aqueuse d'acide alginique avec agitation pour obtenir une solution uniforme et en gouttant la solution uniforme sur un sel de calcium.

2. Procédé pour produire du xylitol à partir de xylose, comprenant :
la préparation d'un milieu de fermentation produisant du xylitol contenant du xylose et du saccharose ;
l'inoculation de la perle d'alginate de la revendication 1 sur le milieu de fermentation ; et
la fermentation du milieu de fermentation dans un fermenteur.

3. Procédé selon la revendication 2, comprenant en outre :
la purification avec une colonne de charbon actif d'une culture de fermentation contenant du xylitol produite par la fermentation ;
la purification avec une colonne d'anions d'une solution de xylitol produite par la purification avec la colonne de charbon actif ; et
la préparation d'une poudre de xylitol à partir d'une solution de xylitol produite par la purification avec la colonne d'anions.
